# EUROPEAN PATENT APPLICATION

(11) **EP 4 542 572 A1**
(43) Date of publication of application: **23.04.2025**
(21) Application number: 23843346.0
(22) Date of filing: 19.07.2023
(51) Int. Cl.: G16H 50/20, G16H 50/50, A61B 5/00, G16H 10/60

(54) **METHOD, PROGRAM, AND APPARATUS FOR PREDICTING HEALTH STATE USING ELECTROCARDIOGRAM SEGMENTS**

(30) Priority: 22.07.2022 KR 20220090764; 18.07.2023 KR 20230092995
(71) Applicant: Medical AI Co., Ltd., Seoul 06180 (KR)
(72) Inventor: KWON, Joonmyoung, Seoul 06180 (KR); JO, Yongyeon, Seoul 06180 (KR)
(74) Representative: Patentanwaltskanzlei Matschnig & Forsthuber OG
(86) International application number: PCT/KR2023/010344
(87) International publication number: WO 2024/019500

(57) **Abstract**

According to an embodiment of the present disclosure, disclosed are a method, a program, and an apparatus, for predicting a health state using electrocardiogram segments. The method may comprise the steps of: dividing electrocardiogram data to generate a plurality of segments; inputting the plurality of segments into a pre-trained machine-learning model to calculate the probabilities of diseases respectively corresponding to the plurality of segments; removing outliers from the probabilities of diseases respectively corresponding to the plurality of segments; and combining the probabilities of diseases from which the outliers have been removed, to generate a result value for prediction of a health state.

## Description

### [Technical Field]

The present disclosure relates to a technology for data processing in the field of medicine, and more particularly, to a method of predicting a health state based on data obtained by filtering electrocardiogram data for stable and accurate analysis of an electrocardiogram using artificial intelligence.

### [Background Art]

An electrocardiogram is a test that records electrical activity of a heart. The electrocardiogram is a relatively simple and cost-effective test method whereby a health state of a heart may be checked, and plays an important role in the early diagnosis and management of heart disease. For example, an electrocardiogram signal measured through an electrocardiogram test may be used to check whether each part of the heart is functioning normally, whether a size and location of the heart are normal, and whether there is damage to a heart muscle, etc. In addition, by checking these things, an electrocardiogram signal may be used to diagnose various heart-related problems and predict a person's health state.

Meanwhile, as artificial intelligence technology develops, attempts to analyze an electrocardiogram using artificial intelligence technology are increasing. One representative example is a service that inputs an electrocardiogram to a neural network-based model and predicts various heart diseases. Most of these conventional technologies and services process all or a part of an electrocardiogram at once through an artificial intelligence model. However, such conventional forms are highly likely to fail to output accurate result values when there is a problem with the electrocardiogram signal itself. For example, when the electrocardiogram signal itself includes much noise or a missing section occurs during the electrocardiogram signal measurement process, the conventional technologies and services may not provide accurate analysis values through the artificial intelligence model and inevitably require the electrocardiogram signal itself to be measured again.

### [Disclosure]

### [Technical Problem]

The present disclosure provides a method capable of increasing accuracy of analysis and convenience of analysis by dividing one electrocardiogram signal into several signals and filtering signals appropriate for analysis.

However, the problems to be solved by the present disclosure are not limited to the problems described above, and other problems that are not mentioned may be clearly understood based on the description below.

### [Technical Solution]

According to an embodiment of the present disclosure, a method of predicting a health state using an electrocardiogram segment, which is performed by a computing device is disclosed. The method includes: inputting the plurality of segments to a pre-trained machine learning model to calculate possibilities of diseases corresponding to each of the plurality of segments; removing outliers from among the possibilities of the diseases corresponding to each of the plurality of segments; and combining the possibilities of the diseases with the outliers removed to generate a result value of the prediction of the health state.

The dividing of the electrocardiogram data to generate the plurality of segments may include dividing a signal in the electrocardiogram data to have a predetermined length without an overlapping area to generate the plurality of segments.

The dividing of the electrocardiogram data to generate the plurality of segments may include dividing the electrocardiogram data while moving a fixed-size window by a preset area in the electrocardiogram data to generate the plurality of segments.

The method may further include inputting the plurality of segments to the pre-trained machine learning model to calculate uncertainty of output corresponding to each of the plurality of segments.

The machine learning model may include a neural network having a probability distribution of neural network weights to quantify the uncertainty of the output.

The removing of the outliers from among the possibilities of the diseases corresponding to each of the plurality of segments may include considering and removing the possibility of the disease corresponding to the uncertainty of the output that is greater than or equal to a first preset reference value, when the uncertainty of the output is greater than or equal to the first preset reference value.

The combining of the possibilities of the diseases with the outliers removed to generate the result value of the prediction of the health state may include comparing an average value of the possibilities of the diseases with the outliers removed with a preset second reference value to generate the result value of the prediction of the health state.

The combining of the possibilities of the diseases with the outliers removed to generate the result value of the prediction of the health state may include comparing each of the possibilities of the diseases with the outliers removed with a preset third reference value to generate the result value of the prediction of the health state.

The comparing of each of the possibilities of the diseases with the outliers removed with the preset third reference value to generate the result value of the prediction of the health state may include: converting the possibilities of the diseases with the outliers removed into binary values based on whether the possibilities of the diseases with the outliers removed are greater than or equal to the preset third reference value; and generating the result value of the prediction of the health state based on a value having a highest ratio among the converted binary values.

According to an embodiment of the present disclosure, a computer program stored in a computer-readable storage medium is disclosed. The computer program performs operations for predicting a health state using an electrocardiogram segment when executed by one or more processors. The operations includes: dividing electrocardiogram data to generate a plurality of segments; inputting the plurality of segments to a pre-trained machine learning model to calculate possibilities of diseases corresponding to each of the plurality of segments; removing outliers from among the possibilities of the diseases corresponding to each of the plurality of segments; and combining the possibilities of the diseases with the outliers removed to generate a result value of the prediction of the health state.

According to an embodiment of the present disclosure, a computing device for predicting a health state using an electrocardiogram segment is disclosed. The device includes: a processor including at least one core; a memory including program codes executable in the processor; and a network unit through which electrocardiogram data is acquired, The processor may divide the electrocardiogram data to generate a plurality of segments, input the plurality of segments to a pre-trained machine learning model to calculate possibilities of diseases corresponding to each of the plurality of segments, remove outliers from among possibilities of diseases corresponding to each of the plurality of segments, and combine the possibilities of the diseases with the outliers removed to generate a result value of the prediction of the health state.

### [Advantageous Effects]

According to a method of the present disclosure, by dividing one electrocardiogram signal into several signals and filtering signals appropriate for analysis, it is possible to increase the accuracy of analysis.

In addition, by dividing one electrocardiogram into several electrocardiograms and filtering signals appropriate for analysis, it is possible to minimize the inconvenience of re-measuring signals required for analysis and increase the convenience of analysis.

### [Description of Drawings]

FIG. 1 is a block diagram of a computing device according to an embodiment of the present disclosure.
FIG. 2 is a block diagram illustrating a process of predicting a health state according to an embodiment of the present disclosure.
FIG. 3 is a conceptual diagram illustrating a neural network structure included in a machine learning model according to an embodiment of the present disclosure.
FIG. 4 is a flowchart illustrating a method of predicting a health state using an electrocardiogram segment according to an embodiment of the present disclosure.

### [Best Mode]

Hereinafter, embodiments of the present disclosure will be described in detail with reference to the attached drawings so that those skilled in the art can easily implement the present disclosure. The embodiments presented in the present disclosure are provided so that those skilled in the art may utilize or implement contents of the present disclosure. Accordingly, various modifications to the embodiments of the present disclosure will be apparent to those skilled in the art. That is, the present disclosure may be implemented in various different forms and is not limited to embodiments described below.

Throughout the specification of the present disclosure, the same or similar drawing symbols refer to the same or similar components. In addition, in order to clearly describe the present disclosure, drawing symbols of parts that are not related to the description of the present disclosure may be omitted in the drawings.

The term "or" used in the present disclosure is intended to mean an inclusive "or," not an exclusive "or." That is, unless otherwise specified in the present disclosure or the meaning is clear from the context, "X uses A or B" is intended to mean one of the natural inclusive substitutions. For example, unless otherwise specified in the present disclosure or the meaning is clear from the context, "X utilizes A or B" may be construed as one of X utilizing A, X utilizing B, or X utilizing both A and B.

The term "and/or" used in the present disclosure should be understood to refer to and include all possible combinations of one or more of the related concepts listed.

The terms "include" and/or "including" used in the present disclosure should be understood to mean that specific features and/or components are present. However, the terms "include" and/or "including" should be understood as not excluding the presence or addition of one or more other features, components and/or groups thereof.

In addition, unless otherwise specified in the present disclosure or the context clearly indicates a singular form, the singular form should generally be construed to include "one or more."

The term "N^{th} (N is a natural number)" used in the present disclosure may be understood as an expression used to distinguish components of the present disclosure according to a predetermined standard such as a functional perspective, a structural perspective, or convenience of description. For example, components performing different functional roles in the present disclosure may be distinguished as a first component or a second component. However, components that are substantially the same within the technical idea of the present disclosure but should be distinguished for convenience of description may also be distinguished as a first component or a second component.

The term "acquisition" used in the present disclosure may be understood to mean not only receiving data through a wired or wireless communication network with an external device or system, but also generating data in an on-device form.

Meanwhile, the term "module" or "unit" used in the present disclosure may be understood as a term referring to an independent functional unit that processes computing resources, such as a computer-related entity, firmware, software or a part thereof, hardware or a part thereof, or a combination of software and hardware. In this case, the "module" or "unit" may be a unit composed of a single element or may be a unit expressed as a combination or set of multiple elements. For example, as a narrow concept, "module" or "unit" may refer to hardware elements of a computing device or a set thereof, an application program that performs a specific function of software, a processing process implemented through software execution, a set of instructions for program execution, etc. In addition, as a broad concept, "module" or "unit" may refer to a computing device itself that constitutes a system, an application that is executed on the computing device, etc. However, the above-described concepts are only examples, and the concept of "module" or "unit" may be variously defined within a category understandable to those skilled in the art based on the contents of the present disclosure.

The term "model" used in the present disclosure may be understood as a system implemented using mathematical concepts and language to solve a specific problem, a set of software units to solve a specific problem, or an abstraction model of a processing process to solve a specific problem. For example, a neural network "model" may be the entire system implemented as a neural network that has a problem-solving ability through learning. In this case, the neural network may obtain its problem-solving ability by optimizing parameters connecting nodes or neurons through learning. The neural network "model" may include a single neural network or may include a neural network set that combines a plurality of neural networks.

The description of the above-described terms is intended to help understanding of the present disclosure. Therefore, when the above-described terms are not explicitly described as matters limiting the contents of the present disclosure, it should be noted that they are not used in a sense that limits the technical ideas of the contents of the present disclosure.

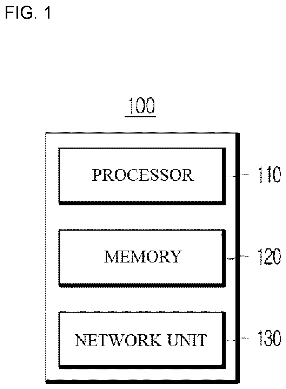

FIG. 1 is a block configuration diagram of a computing device according to an embodiment of the present disclosure.

A computing device 100 according to the embodiment of the present disclosure may be a hardware device or a part of a hardware device that performs comprehensive processing and calculation of data or may be a software-based computing environment connected to a communication network. For example, the computing device 100 may be a server that performs intensive data processing functions and shares resources or may be a client that shares resources through interaction with a server. **In** addition, the computing device 100 may be a cloud system in which a plurality of servers and clients interact to comprehensively process data. Since the above description is only one example related to the type of the computing device 100, the type of the computing device 100 may be configured in various ways within a category understandable to those skilled in the art based on the contents of the present disclosure.

Referring to FIG. 1, the computing device 100 according to the embodiment of the present disclosure may include a processor 110, a memory 120, and a network unit 130. However, FIG. 1 is only one example, and the computing device 100 may include other configurations for implementing a computing environment. In addition, only some of the configurations disclosed above may be included in the computing device 100.

The processor 110 according to the embodiment of the present disclosure may be understood as a configuration unit including hardware and/or software for performing computing operations. For example, the processor 110 may read a computer program and perform data processing for machine learning. The processor 110 may process computational processes such as processing input data for machine learning, feature extraction for machine learning, and error calculation based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA), etc. The type of the processor 110 described above is only one example, and thus the type of the processor 110 may be configured in various ways within a category understandable to those skilled in the art based on the contents of the present disclosure.

The processor 110 may divide electrocardiogram data including an electrocardiogram signal to generate a plurality of segments. The processor 110 may divide one electrocardiogram data into a preset number of arbitrary or fixed sizes to variously generate segments including a portion of the electrocardiogram signal included in the electrocardiogram data. For example, when the electrocardiogram data including the electrocardiogram signal measured for t minutes is acquired, the processor 110 may divide the electrocardiogram data including the electrocardiogram signal measured for t minutes so that segments including an electrocardiogram signal of length i minutes (i is a value smaller than t) are generated.

The processor 110 may input a plurality of segments to a pre-trained neural network model and output possibilities of diseases corresponding to each of the plurality of segments. In this case, the possibility of a disease may include an occurrence probability value of the disease derived by analyzing a portion of the electrocardiogram signal included in the segment. The processor 110 may input a plurality of segments to a pre-trained neural network model and calculate uncertainty of output corresponding to each of the plurality of segments. In this case, the uncertainty of the output may be understood as an indicator of how much the possibility of disease produced by the neural network model may be trusted. That is, the processor 110 may input the segments into the pre-trained neural network model and derive a probability value that a subject whose electrocardiogram signal is being measured has a disease based on each segment. In addition, the processor 110 may confirm the reliability of the derived probability value through the output of the neural network model.

Meanwhile, the neural network model that calculates the possibility of a disease and the uncertainty of the output may perform learning by comparing each output and label through a loss function to adjust the neural network parameters. In this case, a function such as cross entropy may be used as the loss function. The neural network model may perform learning by adjusting neural network parameters in a direction that minimizes the error between the output and the label. The neural network model may perform learning in the form of supervised learning or may perform learning in the form of unsupervised learning, self-supervised learning, etc., depending on the structure of the neural network.

The processor 110 may filter the possibilities of diseases corresponding to each of the plurality of segments based on the uncertainty of the output calculated through the neural network model. The processor 110 may select a value to be analyzed among the possibilities of diseases corresponding to each of the plurality of segments by comparing the uncertainty of the output calculated through the neural network model with a preset reference value. Even in a single electrocardiogram signal, noise or missing values may occur in some sections during the measurement process, and a section where noise or missing values occur may cause a problem that reduces the accuracy of the analysis. Therefore, in order to increase the accuracy of the analysis, the processor 110 may analyze the reliability of the calculated value of each of the plurality of segments and select values with high reliability compared to the reference value as the analysis target.

The processor 110 may combine the filtered possibilities of diseases based on the uncertainty of the output to generate result values of prediction of a health state. The processor 110 may combine the possibilities of diseases corresponding to some sections of the electrocardiogram signal to derive the possibilities of diseases identified from the entire electrocardiogram signal. In addition, the processor 110 may generate results of analyzing the health state the subject whose electrocardiogram signal is being measured based on the possibilities of diseases identified from the entire electrocardiogram signal. In this case, the result of analyzing the health state of the subject may include the diagnosis or prediction results of diseases, such as what disease the subject is currently suffering from or what the probability of occurrence of a disease in the future is. In this way, the processor 110 may obtain more accurate result values than when the entire electrocardiogram signal is not analyzed at once by an operation of dividing the electrocardiogram signal into several signals, performing an in-depth analysis on the divided electrocardiogram signal, and then selecting the analysis values.

The memory 120 according to the embodiment of the present disclosure may be understood as a configuration unit including hardware and/or software for storing and managing data processed in the computing device 100. That is, the memory 120 may store any type of data generated or determined by the processor 110 and any type of data received by the network unit 130. For example, the memory 120 may include at least one of storage media such as a flash memory type, a hard disk type, a multimedia card micro type, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc. In addition, the memory 120 may include a database system that controls and manages data in a predetermined system. Since the types of the memory 120 described above are only examples, the type of the memory 120 may be configured in various ways within a category understandable to those skilled in the art based on the contents of the present disclosure.

The memory 120 may structure, organize, and manage data necessary for performing an operation, combinations of the data, program codes executable by the processor 110, etc. For example, the memory 120 may store medical data received through the network unit 130 to be described below. The memory 120 may store a program code that operates a neural network model to receive medical data and perform learning, a program code that operates the neural network model to receive the medical data and perform inference according to the purpose of use of the computing device 100, and processed data generated as the program codes are executed, etc.

The network unit 130 according to the embodiment of the present disclosure may be understood as a configuration unit that transmits and receives data through any known wired/wireless communication system. For example, the network unit 130 may transmit and receive data using wired or wireless communication systems such as a local area network (LAN), Wideband Code Division Multiple Access (WCDMA), Long Term Evolution (LTE), wireless broadband internet (WiBro), 5th generation mobile communication (5G), ultra wide-band, ZigBee, radio frequency (RF) communication, a wireless LAN, Wi-Fi, near field communication (NFC), or Bluetooth. Since the above-described communication systems are only examples, the wired and wireless communication system for data transmission and reception of the network unit 130 may be applied in various ways other than the above-described examples.

The network unit 130 may receive data necessary for the processor 110 to perform operations through the wired or wireless communication with any system or any client, etc. **In** addition, the network unit 130 may transmit data generated by the operation of the processor 110 through the wired or wireless communication with any system, any client, etc. For example, the network unit 130 may receive medical data through communication with a cloud server, clients such as a smart watch, or medical computing devices, etc., that perform tasks such as databasing within a hospital environment and standardization of medical data. The network unit 130 may transmit output data of the neural network model and intermediate data, processed data, etc., derived from the operation process of the processor 110 through the communication with the above-described database, server, client, computing device, etc.

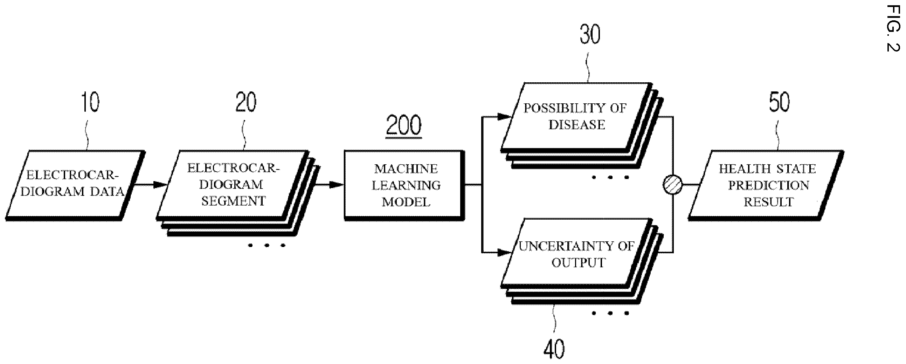

FIG. 2 is a block diagram illustrating a process of predicting a health state according to an embodiment of the present disclosure.

Referring to FIG. 2, the computing device 100 according to the embodiment of the present disclosure may generate electrocardiogram segments 20 including some sections of the electrocardiogram signal based on the electrocardiogram data 10 including the electrocardiogram signal. The computing device 100 may divide the signal from the electrocardiogram data 10 to have a predetermined length without an overlapping area to generate the plurality of electrocardiogram segments 20. The computing device 100 may divide the electrocardiogram data 10 while moving a fixed-size window by a preset area in the electrocardiogram data 10 to generate the plurality of electrocardiogram segments 20.

For example, it is assumed that the computing device 100 has acquired electrocardiogram data including an electrocardiogram signal composed of [A1, A2, A3, A4, A5, A6, A7, A8, A9]. When three segments are generated, the computing device 100 may divide the electrocardiogram data into [A1, A2, A3], [A4, A5, A6], and [A7, A8, A9] so that there is no overlapping area in the entire signal. In addition, when the plurality of segments are generated by moving a window with a size of 3 by one area on the electrocardiogram signal, the computing device 100 may divide the electrocardiogram signal so that some sections overlap, such as [A1, A2, A3], [A2, A3, A4], [A3, A4, A5], and [A7, A8, A9]. These segmentation methods may be preset and selectively performed according to the purpose of use.

The computing device 100 may input the plurality of electrocardiogram segments 20 into the pre-trained machine learning model 200 to derive a possibility 30 of a disease and an uncertainty 40 of output for each of the plurality of segments 20. Specifically, the computing device 100 may input each of the plurality of segments 20 to the machine learning model 200 to derive the possibility 30 of a disease including probability values of occurrence of diseases corresponding to each of the plurality of segments. In addition, the computing device 100 may acquire the uncertainty 40 of output corresponding to each of the possibilities 30 of diseases from the output of the machine learning model 200.

For example, it is assumed that the plurality of segments are generated as S1, S2, S3, and Sn. In this case, each of the plurality of segments has information on a certain size (or time). The computing device 100 may input S1 to the machine learning model and output P1 corresponding to the possibility of a disease analyzed from S1. In addition, the computing device 100 may also obtain U1, which is uncertainty indicating how much the value P1 may be trusted, from the output of the machine learning model. The computing device 100 may input S2 to the machine learning model and output P2 corresponding to a possibility of a disease analyzed from S2. In addition, the computing device 100 may also obtain U1, which is uncertainty indicating how much the value P1 may be trusted, from the output of the machine learning model. The computing device 100 may sequentially repeat this process to calculate Pn corresponding to the possibility of a disease analyzed from Sn and Un indicating the reliability therein.

The computing device 100 may remove outliers existing in the possibility 30 of a disease corresponding to each of the plurality of segments based on the uncertainty 40 of output. The computing device 100 may compare the uncertainty 40, which is a pair with the possibility 30 of a disease, with a preset reference value, and extract outliers that are determined to be difficult to trust as analysis values within the possibility 30 of a disease. Through the outlier removal (or extraction) process, the computing device 100 may extract the basis for deriving the result 50 of the prediction of the health state as high-quality data.

For example, the computing device 100 may compare the uncertainty U1 indicating the degree of confidence of the possibility of a disease P1 with a preset first reference value, and determine whether U1 is greater than or equal to the first reference value. When U1 is greater than or equal to the first reference value, the computing device 100 may consider P1 corresponding to U1 as an outlier and remove P1. When U1 is smaller than or equal to the first reference value, the computing device 100 may consider P1 corresponding to U1 as an outlier and remove P1. The computing device 100 may determine and filter whether Pn is an outlier by sequentially repeating the above-described process.

The computing device 100 may combine the possibilities of diseases with the outliers removed to generate the result 50 of the prediction of the health state. The computing device 100 may generate the result 50 of the prediction of the health state by comparing the average value of the possibilities of diseases with the outliers removed with a preset second reference value. The computing device 100 may also generate the result 50 of the prediction of the health state by comparing each possibility of a disease with the outliers removed with a preset third reference value.

For example, it is assumed that the outlier is removed through the above process and the remaining possibility of a disease is three values such as 0.5, 0.7, and 0.4. The computing device 100 may calculate the average of the three values, (0.5+0.7+0.4)/3 = 0.53. In this case, when the second reference value is set to 0.5, because the average of the three values, 0.53, is greater than the second reference value, 0.51, the computing device 100 may finally determine that a specific disease has occurred.

In addition, when the third reference value is set to 0.51, the computing device 100 can compare the third reference value, 0.51, with each of the three values. Since the first value, 0.5 is smaller than the third reference value, 0.51, the computing device 100 may tag 0, which indicates that the specific disease has not occurred, to the value. Since the second value, 0.7 is greater than the third reference value, 0.51, the computing device 100 may tag 1, which indicates that the specific disease has occurred, to the corresponding value. Since the third value, 0.4 is smaller than the third reference value, 0.51, the computing device 100 may tag 0 to the corresponding value. The computing device 100 may finally determine that the specific disease has not occurred based on 0 which is a value that accounts for the largest proportion among values tagged as 0, 1, and 0. This final determination result may be reflected in the result of the prediction of the health state.

Referring to the examples described above, it can be seen that the results of comparing the second reference value with the average value and comparing the third reference value with the individual value may be different. Therefore, which method is used may be determined based on the user's selection according to their intended purpose. In other words, the computing device 100 may determine how to perform the final determination based on the user input.

Meanwhile, the first reference value, the second reference value, or the third reference value may be a value preset by the user depending on the purpose of use. For example, when the disease analyzed through the machine learning model is a rare disease, the possibility of misdiagnosis may be higher compared to other diseases when the uncertainty of the analysis result is high. Therefore, in such cases, the first reference value, the second reference value, or the third reference value may be set higher compared to other diseases. In this way, the reference value may be set in various ways depending on the type of disease, the attributes of data, etc.

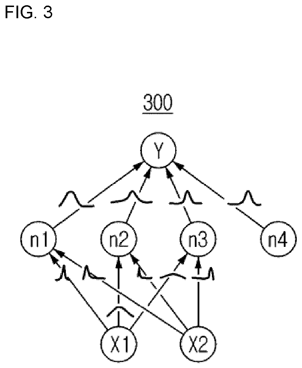

FIG. 3 is a conceptual diagram illustrating a neural network structure included in a machine learning model according to an embodiment of the present disclosure.

Referring to FIG. 3, a machine learning model according to an embodiment of the present disclosure may include a neural network 300 having a probability distribution of neural network weights in order to quantify the uncertainty of output. The neural network 300 of FIG. 3 assumes that the neural network weights do not exist as fixed values but exist as the probability distribution. That is, the neural network 300 of FIG. 3 may assume that the trained neural network weights follow a Gaussian distribution. In addition, in the training process of the neural network 300 of FIG. 3, the neural network weight values themselves are not adjusted, but the average and variance of the Gaussian distribution may be adjusted. The machine learning model may derive uncertainty corresponding to an indicator of how reliable the possibility of disease, which is the output value, is through such a neural network 300.

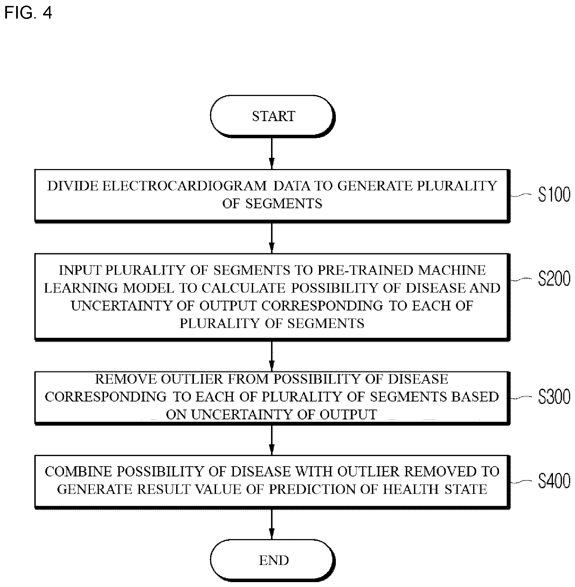

FIG. 4 is a flowchart illustrating a method of predicting a health state using an electrocardiogram segment according to an embodiment of the present disclosure.

Referring to FIG. 4, the computing device 100 according to the embodiment of the present disclosure may divide the electrocardiogram data to generate the plurality of segments (S100). When the computing device 100 is a server providing an electrocardiogram reading service, the computing device 100 may acquire electrocardiogram data by communicating with a device that measures an electrocardiogram signal. When the computing device 100 is a device that measures and analyzes the electrocardiogram signal, the computing device 100 may measure the electrocardiogram signal through a measuring unit to generate the electrocardiogram data. When the electrocardiogram data is acquired, the computing device 100 may divide the signal in the electrocardiogram data to have a predetermined length without an overlapping area to generate the plurality of segments. The computing device 100 may also divide the electrocardiogram data while moving a fixed-size window by a preset area in the electrocardiogram data to generate the plurality of segments.

The computing device 100 may input the plurality of segments to the pre-trained machine learning model, and calculate the possibility of a disease and the uncertainty of output corresponding to each of the plurality of segments (S200).

The computing device 100 may remove the outliers from among the possibilities of diseases corresponding to each of the plurality of segments based on the uncertainty of output (S300). The computing device 100 may compare the uncertainty of output with the preset first reference value. When the uncertainty of output is greater than or equal to the preset first reference value, the computing device 100 may consider and remove the possibility of a disease corresponding to the uncertainty of output greater than or equal to the first reference value as the outlier. When the uncertainty of output is less than or equal to the preset first reference value, the computing device 100 may use the possibility of a disease corresponding to the uncertainty of output less than the first reference value for the final analysis performed in operation S400.

The computing device 100 may combine the possibilities of diseases with the outliers removed to generate the result value of the prediction of the health state (S400). The computing device 100 may compare the average value of the possibilities of diseases with the outliers removed with the preset second reference value to generate the result value of the prediction of the health state. The computing device 100 may also compare each possibility of a disease with the outliers removed with the preset third reference value to generate the result value of the prediction of the health state. Specifically, the computing device 100 may convert the possibility of a disease with the outliers removed into a binary value based on whether the possibility of a disease with the outliers removed is greater than or equal to the preset third reference value. Then, the computing device 100 may generate the result value of the prediction of the health state based on the value that accounts for the highest ratio among the converted binary values.

Various embodiments of the present disclosure described above may be combined with additional embodiments and can be modified within a range that may be understood by those skilled in the art in light of the detailed description set forth above. The embodiments of the present disclosure are illustrative in all respects and should be understood as non-limiting. For example, each component described as a single type may be implemented in a distributed manner, and similarly, components described as distributed may be implemented in a combined form. Accordingly, all changes or modifications derived from the meaning, scope, and equivalent concept of the claims of the present disclosure should be construed as being included in the scope of the present disclosure.

## Claims

1. A method of predicting a health state using an electrocardiogram segment, which is performed by a computing device including at least one processor, the method comprising:
dividing electrocardiogram data to generate a plurality of segments;
inputting the plurality of segments to a pre-trained machine learning model to calculate possibilities of diseases corresponding to each of the plurality of segments;
removing outliers from among the possibilities of the diseases corresponding to each of the plurality of segments; and
combining the possibilities of the diseases with the outliers removed to generate a result value of the prediction of the health state.

2. The method of claim 1, wherein the dividing of the electrocardiogram data to generate the plurality of segments includes dividing a signal in the electrocardiogram data to have a predetermined length without an overlapping area to generate the plurality of segments.

3. The method of claim 1, wherein the dividing of the electrocardiogram data to generate the plurality of segments includes dividing the electrocardiogram data while moving a fixed-size window by a preset area in the electrocardiogram data to generate the plurality of segments.

4. The method of claim 1, further comprising inputting the plurality of segments to the pre-trained machine learning model to calculate uncertainty of output corresponding to each of the plurality of segments.

5. The method of claim 4, wherein the machine learning model includes a neural network having a probability distribution of neural network weights to quantify the uncertainty of the output.

6. The method of claim 4, wherein the removing of the outliers from among the possibilities of the diseases corresponding to each of the plurality of segments includes considering and removing the possibility of the disease corresponding to the uncertainty of the output that is greater than or equal to a first preset reference value, when the uncertainty of the output is greater than or equal to the first preset reference value.

7. The method of claim **1,** wherein the combining of the possibilities of the diseases with the outliers removed to generate the result value of the prediction of the health state includes comparing an average value of the possibilities of the diseases with the outliers removed with a preset second reference value to generate the result value of the prediction of the health state.

8. The method of claim 1, wherein the combining of the possibilities of the diseases with the outliers removed to generate the result value of the prediction of the health state includes comparing each of the possibilities of the diseases with the outliers removed with a preset third reference value to generate the result value of the prediction of the health state.

9. The method of claim 8, wherein the comparing of each of the possibilities of the diseases with the outliers removed with the preset third reference value to generate the result value of the prediction of the health state includes:
converting the possibilities of the diseases with the outliers removed into binary values based on whether the possibilities of the diseases with the outliers removed are greater than or equal to the preset third reference value; and
generating the result value of the prediction of the health state based on a value having a highest ratio among the converted binary values.

10. A computer program that is stored in a computer-readable storage medium and performs operations for predicting a health state using an electrocardiogram segment when executed by one or more processors, wherein the operations include:
dividing electrocardiogram data to generate a plurality of segments;
inputting the plurality of segments to a pre-trained machine learning model to calculate possibilities of diseases corresponding to each of the plurality of segments;
removing outliers from among the possibilities of the diseases corresponding to each of the plurality of segments; and
combining the possibilities of the diseases with the outliers removed to generate a result value of the prediction of the health state.

11. A device that is a computing device for predicting a health state using an electrocardiogram segment, comprising:
a processor including at least one core;
a memory including program codes executable in the processor; and
a network unit through which electrocardiogram data is acquired,
wherein the processor divides the electrocardiogram data to generate a plurality of segments,
inputs the plurality of segments to a pre-trained machine learning model to calculate possibilities of diseases corresponding to each of the plurality of segments,
removes outliers from among possibilities of diseases corresponding to each of the plurality of segments, and
combines the possibilities of the diseases with the outliers removed to generate a result value of the prediction of the health state.
